# NEW EUROPEAN PATENT SPECIFICATION

(11) **EP 3 516 972 B2**
(45) Date of publication and mention of the opposition decision: **08.04.2026**
(45) Mention of the grant of the patent: 02.12.2020
(21) Application number: 17892066.6
(22) Date of filing: 11.12.2017
(51) Int. Cl.: A24F 47/00, A61M 15/06, A61M 11/04, A24F 40/44

(54) **ELECTRONIC CIGARETTE AND ATOMIZER THEREOF**
ELEKTRONISCHE ZIGARETTE UND ZERSTÄUBER DAFÜR
CIGARETTE ÉLECTRONIQUE ET ATOMISEUR ASSOCIÉ

(43) Date of publication of application: 31.07.2019
(73) Proprietor: Shenzhen Smoore Technology Limited, Shenzhen, Guangdong 518102 (CN)
(72) Inventor: PAN, Weidong, Shenzhen, Guangdong 518102 (CN)
(74) Representative: Erdmann, Matthias
(86) International application number: PCT/CN2017/115486
(87) International publication number: WO 2019/113747

(56) References cited:
- EP-A1- 3 287 017
- EP-A1- 3 287 017
- WO-A1-2015/077645
- WO-A1-2016/161554
- WO-A1-2017/048782
- WO-A1-2017/143865
- WO-A2-2014/089283
- CN-A- 103 960 782
- CN-A- 104 720 117
- CN-A- 104 824 853
- CN-A- 105 559 147
- CN-A- 105 705 048
- CN-A- 106 418 709
- CN-A- 106 820 271
- CN-U- 203 523 811
- CN-U- 204 409 592
- CN-U- 204 409 592
- CN-U- 205 695 694
- CN-U- 205 865 992
- CN-U- 206 119 186
- CN-U- 206 565 287
- US-A- 5 160 518
- US-A1- 2015 208 729
- US-A1- 2016 007 655
- US-A1- 2016 095 354
- US-A1- 2017 215 481

## Description

### TECHNICAL FIELD

The present disclosure relates to the technical field of smoking device, particularly relates to an electronic cigarette and an atomizer thereof.

### BACKGROUND

An electronic cigarette is also known as a virtual cigarette. Since an electronic cigarette has the similar appearance and taste of cigarette, but generally does not contain harmful ingredients, such as tar and particulate matter, it is widely welcomed by users.

An atomizer is a key device in an electronic cigarette, which is used to store atomizing liquid, and to atomize the atomizing liquid. In a conventional electronic cigarette, the atomizer has a complex structure, and it is time-consuming and laborious to assemble. Examples of known electronic cigarettes are disclosed in documents CN 106 820 271, CN 205 695 694, CN 204 409 592 and CN 104 824 853.

### SUMMARY

Accordingly, it is necessary to provide an electronic cigarette and an atomizer thereof with a simple structure and easy assembly.

In order to solve this problem, according to the invention an atomizer as per appended claim 1 is provided.

An electronic cigarette includes:
an aforementioned atomizer; and
a battery device including a cover, a battery and a pogo pin, the battery is received in the cover, the pogo pin is mounted on the cover, and the pogo pin is electrically connected to the battery and the heating element, respectively.

The details of one or more embodiments of present disclosure are set forth in the accompanying drawings and the description below. Other features, objects, and advantages of present disclosure will be apparent from the description and drawings, and from the claims.

### BRIEF DESCRIPTION OF THE DRAWINGS

To illustrate the technical solutions according to the embodiments of the present disclosure or in the prior art more clearly, the accompanying drawings for describing the embodiments or the prior art are introduced briefly in the following. Apparently, the accompanying drawings in the following description are only some embodiments of the present disclosure, and persons of ordinary skill in the art can derive other drawings from the accompanying drawings without creative efforts.
FIG. 1 is a schematic view of an electronic cigarette according to an embodiment;
FIG. 2 is an exploded view of an atomizer of the electronic cigarette of FIG. 2;
FIG. 3 is a cross-sectional view of the atomizer of the electronic cigarette of FIG. 2;
FIG. 4 is another cross-sectional view of the atomizer of the electronic cigarette of FIG. 2;
FIG. 5 is a cross-sectional view of a heating assembly of the atomizer of FIG. 4; and
FIG. 6 is cross-sectional view of a battery device of the electronic cigarette of FIG. 2.

### DETAILED DESCRIPTION OF THE EMBODIMENTS

Embodiments of the present disclosure are described more fully hereinafter with reference to the accompanying drawings. A preferred embodiment is described in the accompanying drawings. The various embodiments of the invention may, however, be embodied in many different forms and should not be construed as limited to the embodiments set forth herein. Rather, these embodiments are provided so that this disclosure will be thorough and complete, and will fully convey the scope of the invention to those skilled in the art.

Unless otherwise defined, all terms (including technical and scientific terms) used herein have the same meaning as commonly understood by one of ordinary skill in the art to which this invention belongs. The terms used herein is for the purpose of describing particular embodiments only and is not intended to limit the present disclosure. As used herein, the term "and/or" includes any and all combinations of one or more of the associated listed items.

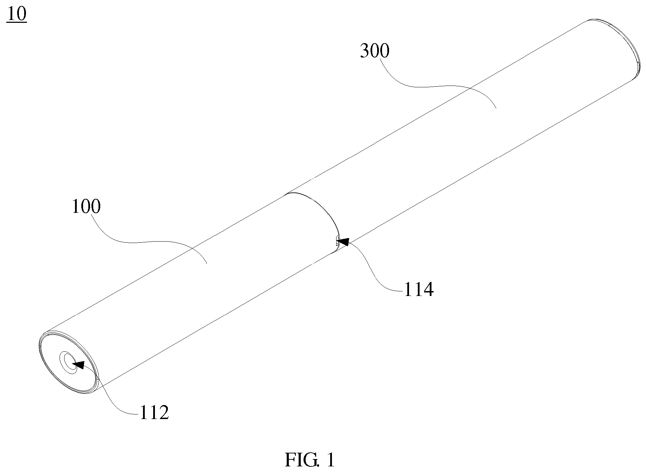

Referring to FIG. 1, an electronic cigarette 10 according to an embodiment includes an atomizer 100 and a battery device 300. The battery device 300 is connected to the atomizer 100, and the battery device 300 is used to power the atomizer 100.

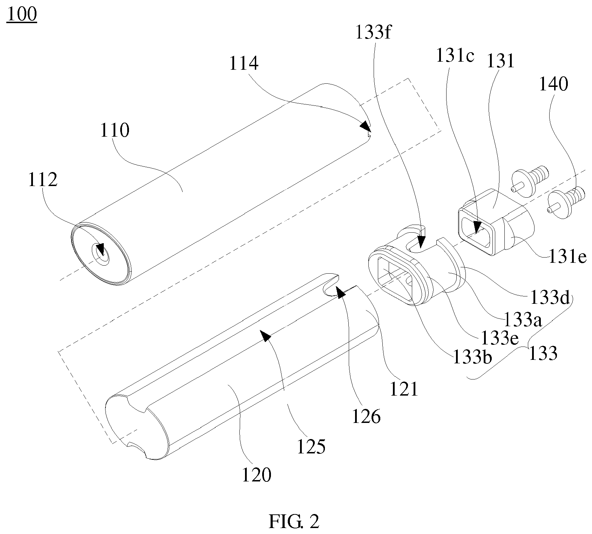

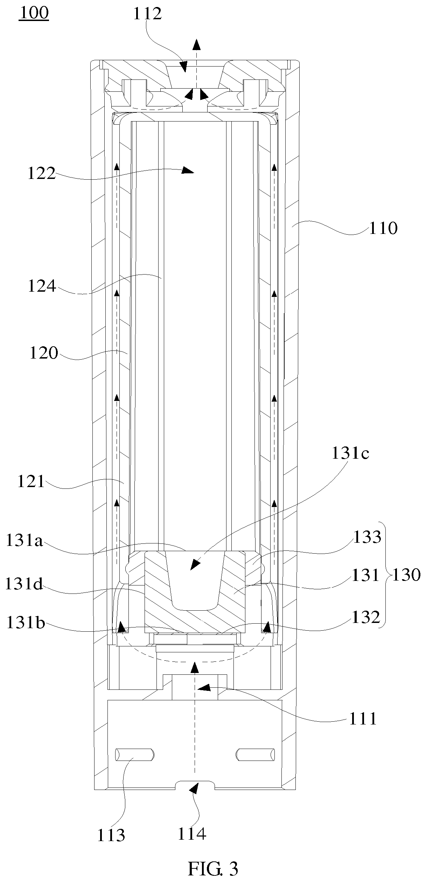

Referring to FIG. 2, FIG. 3, and FIG. 4, the atomizer 100 includes a liquid reservoir 120 and a heating assembly 130. The liquid reservoir 120 has a liquid storage cavity 122 used to receive atomizing liquid, the liquid reservoir 120 also has an opening end 121, and the opening end 121 defines an opening in communication with the liquid storage cavity 122. The liquid reservoir 120 can be a hollow structure of a tubular shape, a cubic shape, a spherical shape or the like that can receive the atomizing liquid. The heating assembly 130 is connected to the liquid reservoir 120, so as to absorb and atomize the atomizing liquid stored in the liquid storage cavity 122.

The heating assembly 130 includes a liquid conducting body 131 and a heating element 132. The liquid conducting body 131 is located on the opening end 121, and the conducting body 131 has a liquid absorbing surface 131a facing an inside of the liquid storage cavity 122, and has an atomizing surface 131b located outside of the liquid storage cavity 122, the liquid conducting body 131 conducts the atomizing liquid in the liquid storage cavity 122 to the atomizing surface 131b. The heating element 132 is formed on the atomizing surface 131b, and the heating element 132 atomizes the atomizing liquid conducted to the atomizing surface 131b, so as to obtain an atomized gas for smoking.

In addition, the liquid absorbing surface 131a defines a recess 131c, and an minimum conduction distance of the atomizing liquid from a bottom wall of the recess 131c to the atomizing surface 131b is less than a minimum conduction distance of the atomizing liquid from the liquid absorbing surface 131b to the atomizing surface 131b. In the illustrated embodiment, the liquid conducting body 131 can be of any shape. The liquid absorbing surface 131a and the atomizing surface 131b are arranged in parallel. When no recess 131c is formed on the liquid absorbing surface 131a, the atomizing liquid has a minimum conduction distance, and when the recess 131c is defined on the liquid absorbing surface 131a, another minimum conduction distance is formed between an bottom wall of the recess 131c and the atomizing surface 131b. Since the minimum conduction distance of the atomizing liquid from the bottom wall of the recess 131c to the atomizing surface 131b is further less than the minimum conduction distance of the atomizing liquid from the liquid absorbing surface 131a to the atomizing surface 131b, the atomizing liquid can enter the recess 131c quickly and travel along the a shortest traveling path formed between the bottom wall of the recess 131c and the atomizing surface 131b, such that a conduction distance of the atomizing liquid is reduced, the resistance subjected during the flow of the atomizing liquid is reduced, and the conduction efficiency of the liquid conducting body 131 is improved. Meanwhile, compared with the solution in which the recess 131c is not formed on the liquid absorbing surface 131a, the liquid conducting body 131 of this solution has a greater contact area with the atomizing liquid, which facilitates the conduction of the atomizing liquid, and also increases the volume of the liquid storage cavity 122, so as to increase the amount of liquid stored in the liquid storage cavity 122.

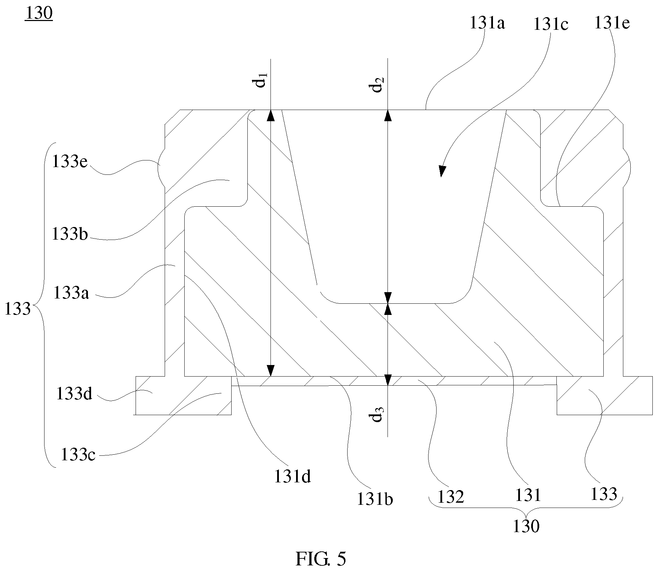

Referring to FIG. 5, the liquid absorbing surface 131a and the atomizing surface 131b are located on opposite sides of the liquid conducting body 131, and the liquid conducting body 131 further includes a side surface 131d connecting the liquid absorbing surface 131a and the atomizing surface 131b. In the illustrated embodiments, the liquid conducting body 131 is of a columnar structure, and the two opposite end surfaces of the liquid conducting surface 131a are the liquid absorption 131a and the atomizing surface 131b, which are arranged in parallel. at this time, a minimum transportation distance d₁ between the liquid absorbing surface 131a and the atomizing surface 131b is the perpendicular distance between the liquid absorbing surface 131a and the atomizing surface 131b. By providing the side surface between the liquid absorbing surface 131a and the atomizing surface 131b, the liquid conducting body 131 has a certain thickness.

In one of the embodiments, the recess 131c extends along a direction from the liquid absorbing surface 131a to the atomizing surface 131b. The recess 131c extends along a linear direction perpendicular to the atomizing surface 131b and approaches the atomizing surface 131b. In this case, a flowing path of the atomizing liquid along the recess 131c is a straight line, therefore this configuration can prevent the problem of a relatively large resistance of the atomizing liquid caused by a longer flowing path of the atomizing liquid along the recess 131c when the recess 131c is curved, so as to shorten the flowing path of the atomizing liquid along the recess 131c, reduce the resistance to the atomizing liquid, and improve a transfer efficiency of the atomizing liquid. Meanwhile, a depth d₂ of the recess 131c is a distance from the bottom wall of the recess 131c to the liquid absorbing surface 131a, and the minimum conduction distance d₃ from the bottom wall of the recess 131c to the liquid absorbing surface is a distance from a position at the bottom wall of the recess 131c farthest from the liquid absorbing surface 131a to the atomizing surface 131b. When the atomizing liquid enters the recess 131c, the atomizing liquid will be conducted from the position at the bottom wall of the recess 131c farthest from the liquid absorbing surface 131a to the atomizing surface 131b.

Additionally, in one of the embodiments, the minimum conduction distance between the bottom wall of the recess 131c and the atomizing 131b is less than the depth of the recess 131c, in this way, the minimum conduction distance d3 between the bottom wall of the recess 131c is further less than the depth d2 of the recess 131c. When the thickness of the liquid conducting body 131 is constant, the minimum conduction distance d3 can be reduced via increasing the depth d2 of the recess 131c, such that the atomizing liquid can flow to a position at the bottom wall of the recess 131c that nearest to the atomizing surface 131b as soon as possible, and the atomizing liquid can be conducted through the minimum conduction distance d3 between the bottom wall of the recess 131c and the atomizing surface 131b to the atomizing surface 131b under a relatively less resistance, so as to improve the conduction efficiency of the liquid conducting body 131.

In one of the embodiments, a cross-sectional area of the recess gradually decreases along a direction from the liquid absorbing surface 131a to the atomizing surface 131b, such that the recess 131c can have a relatively greater opening at the liquid absorbing surface 131a, and the atomizing liquid can enter the recess 131c smoothly without forming a film at the opening of the recess 131c to block the atomizing liquid from flowing along the recess 131c. The recess 131c can have a step shape, a cone shape, or a frustum shape.

According to the invention, the bottom wall of the recess 131c is parallel to the atomizing surface 131b. In this case, the minimum conduction distance between the bottom wall of the recess 131c and the atomizing surface 131b is the distance between the bottom wall of the recess 131c and the atomizing surface 131b. The atomizing liquid can be conducted to the atomizing surface 131b with the minimum conduction distance from any position at the bottom wall of the recess 131c, thus the conduction efficiency of the liquid conducting body 131 is further improved.

In one of the embodiments, the liquid conducting body 131 is a porous body. The liquid conducting body 131 defines a plurality of micropores, and the atomizing liquid can flow along the micropores and be conducted to the atomizing surface 131b. Pore size of the micropores can be adjusted according to the type of the atomizing liquid, for example, if the viscosity of the atomizing liquid is large, a liquid conducting body 131 with greater pore size can be selected, such that the conduction efficiency of the liquid conducting body 131 is moderate. Moreover, in one of the embodiments, the liquid conducting body 131 is a porous ceramic.

In one of the embodiments, the heating element 132 can be a heating coat, a heating circuit, a heating chip, or a heating net. The heating coat can be coated on the atomizing surface 131b. The heating circuit can be plated on the atomizing surface 131b. The heating chip and the heating net can be mounted on the atomizing surface 131b via other auxiliary mounting means. The heating element 132 has a thin layer structure aligned with the atomizing surface 131b, so as to uniformly heat the atomizing surface 131b, such that the temperature for atomizing is consistent to avoid greater atomized particles caused by local lower temperatures, the atomized particles are uniform and the taste of the electronic cigarette is improved. Meanwhile, the heating element 132 has a greater contact area with the atomizing liquid, so as to improve the atomization efficiency.

Referring to FIG. 2 to FIG. 5, in one of the embodiments, the heating assembly 130 further includes a sealing element 133, which is at least partially located between the liquid conducting body 131 and the liquid reservoir 120, so as to prevent the atomizing liquid in the liquid reservoir 120 from directly flowing out without passing through the liquid conducting body 131. Because of the sealing effect of the sealing element 133, the atomizing liquid in the liquid reservoir 120 can only be conducted along the conduction path between the liquid absorbing surface 131a and the atomizing surface 131b. Furthermore, the liquid conducting body 131 is required to have a certain thickness to satisfy a mounting requirement of the sealing element 133, defining the recess 131c on the liquid absorbing surface 131a can make the liquid conducting body 131 not only meet the requirement of thickness, but also meet the requirement of conduction efficiency.

Additionally, in one of the embodiments, a side of the liquid conducting body 131 adjacent to the liquid absorbing surface 131a extends into the opening of the liquid reservoir 120. The sealing element 133 includes a sealing body 133a, which is sleeved on a side surface 131d of the liquid conducting body 131, and abuts against an inner wall of the liquid reservoir 120, so as to seal a gap between the liquid conducting body 131 and the inner wall of the liquid reservoir 120.

In one of the embodiments, the sealing element 133 further includes a first latching portion 133b and a second latching portion 133c. The first latching portion 133b is formed on an end of the sealing body 133a adjacent to the liquid absorption surface 131a, and the second latching portion 133c is formed on an end of the sealing body 133a adjacent to the atomizing surface 131b. The first latching portion 133b and the second latching portion 133c are latched to both ends of the liquid conducting body 131, respectively, so as to limit the relative mounting of the liquid conducting body 131 and the sealing element 133, which not only facilitates the mounting of the liquid conducting body 131 and the sealing element 133, but also prevents the liquid conducting body 131 from separating with the sealing element 133 to ensure an effective sealing of the sealing body 133a. Moreover, in one of the embodiments, the first latching portion 133b and the second latching portion 133c are both formed on an inner wall of the sealing body 133a. A boss portion 131e is formed on the side wall 131d of the liquid conducting body 131, the first latching portion 133b abuts against the boss portion 131e, and the second latching portion 133c abuts against the atomizing surface 131b, so as to limit the position of the liquid conducting body 131.

In one of the embodiments, the sealing element 133 further includes a third latching portion 133d. The third latching portion 133d is formed on an end of an outer wall of the sealing body 133a adjacent to the atomizing surface 131b, and the third latching portion 133d abuts against the end surface of the opening end 121 of the liquid reservoir 120. The configuration of the third latching portion 133d can limit the mounting of the heating assembly 130 at the opening of the liquid reservoir 120, so as to prevent the sealing element 133 and the liquid conducting body 131 from extending into the opening too deep, and to facilitate the mounting of the heating assembly 130 and the liquid reservoir 120.

In one of the embodiments, the sealing element 133 further includes a sealing ring 133e formed on an end of an outer wall of the sealing body 133a adjacent to the liquid absorbing surface. The sealing ring 133e can firmly abut against an inner wall of the liquid reservoir 120, so as to improve the sealing between the liquid conducting body 131 and the liquid reservoir 120.

In one of the embodiments, the sealing element 133 is an integrated structure. The sealing body 133a, the first latching portion 133d, the second latching portion 133c, the third latching portion 133d and the sealing ring 133e are integrally formed. The sealing element 133 can be made of silicone material, or other materials with sealing and thermal insulation features. The sealing element 133 enwraps the liquid conducting body 131, so as to reduce the unnecessary volatilization of the atomizing liquid, and also to achieve a thermal insulation effect. The silicon material can also avoid a hard contact between the liquid conducting body 131 and other parts, so as to avoid a damage of the liquid conducting body 131.

Referring to FIG.2 to FIG4, in one of the embodiments, an side of the liquid conducting body 131 adjacent to the liquid absorbing surface 131a extends into the opening of the liquid reservoir 120. the inner wall of the liquid reservoir 120 is provided with a strengthening rib 124, which is capable of abutting against a side of the heating assembly 130 located in the liquid storage cavity 122. Furthermore, the strengthening rib 124 can abut against the liquid absorbing surface 131a of the liquid conducting body 131, so as to limit a depth of the liquid conducting body 131 extending into the liquid reservoir 120. Alternatively, when the sealing body 133a is sleeved on the side surface 131d of the liquid conducting body 131 and abuts against the inner wall of the liquid reservoir 120, the strengthening rib 124 can also abut against an end of the sealing element 133 adjacent to the liquid absorbing surface 131a, so as to limit a depth of the sealing element 133 extending into the liquid reservoir 120. Alternatively, the strengthening rib 124 can simultaneously abut against end surfaces of the liquid absorbing surface 131a of the liquid conducting body 131 and the sealing element 133, so as to limit a depth of the heating assembly 130 extending into the liquid reservoir 120. According to an embodiment, according to an embodiment, a plurality of the strengthening ribs 124 are provided, and the plurality of strengthening ribs 124 are uniformly distributed along a peripheral wall surface of the liquid reservoir 120, so as to apply a uniform force to the end of the heating assembly 130 located in the liquid storage cavity 122.

In one of the embodiments, the atomizer 100 further includes a housing 110. The housing 110 defines an air inlet 111 and an air outlet 112, the heating assembly 130 and the liquid reservoir 120 are received in the housing 110. The heating assembly 130 is located adjacent to the air inlet 111, and an airflow passage communicating the air inlet 111 and the air outlet 112 is provided between the housing 110 and the liquid reservoir 120. Airflow can enter from the air inlet 111, and the atomized gas obtained via an atomization of the heating element 132 flows along with the airflow, passes through the airflow passage and flows out from the air outlet 112 for smoking.

Moreover, in one of embodiments, the liquid reservoir 120 has a long tubular structure, and an outer wall of the liquid reservoir 120 is slightly less than an inner wall of the housing 110, such that the liquid reservoir 120 can be received in the housing 110. A gap between the outer wall of the liquid reservoir 120 and the inner wall of the housing 110 is relatively small, which is not conducive for the airflow and the atomized gas to be conducted out. A longitudinal groove 125 is defined on the outer wall of the liquid reservoir 120, such that an airflow passage is formed between the liquid reservoir 120 and the housing 110, so as to facilitate the airflow flowing along the longitudinal groove 125 and being conducted to the air outlet 112. Alternatively, the longitudinal groove 125 can be defined on the inner wall of the housing 110. Alternatively, the longitudinal grooves 125 can be simultaneously defined on the outer wall of the liquid reservoir 120 and the inner wall of the housing 110. Moreover, two longitudinal grooves 125 can be defined on the outer wall of the liquid reservoir 120, and the two longitudinal grooves 125 are oppositely arranged, so as to form two symmetrical airflow passages between the liquid reservoir 120 and the housing 110, such that a flux of gas and the atomized gas is increased, and the smoking taste is improved.

In one of the embodiments, a portion of the sealing element 133 is located between the liquid conducting body 131 and the liquid reservoir 120, so as to seal a gap between the liquid reservoir 120 and the liquid conducting body 131. The portion of the sealing element 133 abuts against the inner wall of the housing 110, such that more airflow entering through the air inlet 111 can reach the heating element 132 and is fully mixed with the atomized gas, so as to carry more atomized gas to the air outlet 112. At this time, the sealing element 133 defines a first notch 133f, the opening end 121 of the liquid reservoir 120 defines a second notch 126, and the first notch 133f and the second notch 126 communicate the air inlet 111 and the longitudinal groove 125. The airflow carrying the atomized gas enters the longitudinal groove 125 through the first notch 133f and the second notch 126, and is conducted out through the air outlet 112. Moreover, both of the number of the first notch 133f and the second notch 126 are two, such that two longitudinal grooves 125 can be in communication with the air inlet 111.

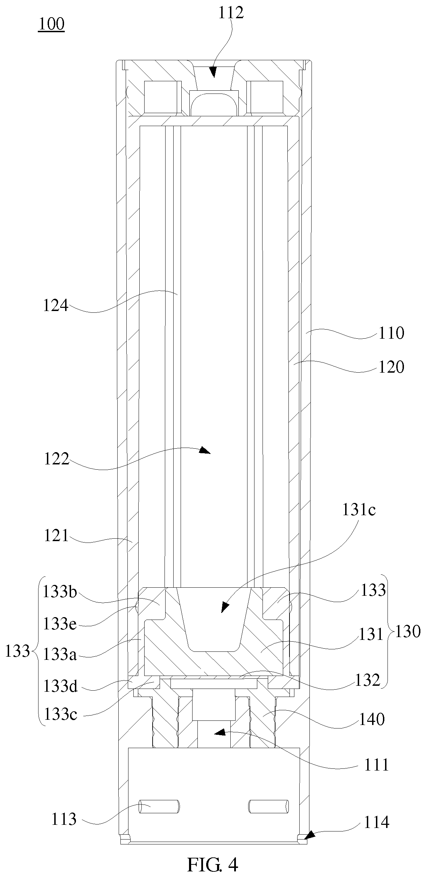

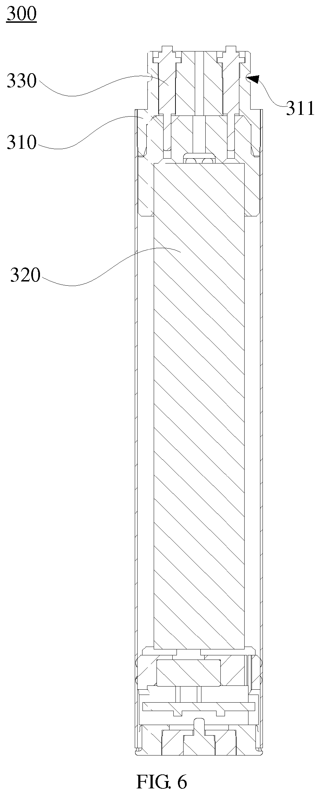

Referring FIG 1 and FIG. 6, in one of the embodiments, the battery device 300 includes a cover 310, a battery 320 and a pogo pin 330. The battery 320 is received in the cover 310, the pogo pin 330 is mounted on the cover 310, and is electrically connected to the battery 320 and the heating element 132, respectively. Referring to FIG. 2 and FIG. 4, in one of the embodiments, the atomizer 100 further includes an electrode 140, the electrode 140 is mounted on the housing 110, and is electrically connected to the heating element 132. The pogo pin 330 is electrically connected to the electrode 140, so as to power the heating element 132 via the electrode 140. In one of the embodiments, the cover 310 latches with the housing 110, so as to achieve a fast dismounting of the battery device 300 and the atomizer 100. Moreover, referring to FIG. 4 and FIG. 6, an end of the cover 310 is received in the housing 110, and the inner wall of the housing 110 is provided with a protruded portion 113, an outer wall of the cover 310 defines a limiting groove 311, and the protruded portion 113 can latches into the limiting groove 311, so as to achieve a fast connection of the housing 110 and the cover 310. Alternatively, an end of the housing 110 can be received in the cover 310. Moreover, the housing defines an inlet notch 114, and a certain gap between the cover 310 and the housing 110 forms an inlet passage, the inlet passage is connected to the inlet 11 of the housing 110, and the airflow passes through the inlet passage between the cover 310 and the housing 110, then enters the housing 110 through the inlet 111.

The technical features of the embodiments described above can be arbitrarily combined. In order to make the description succinct, there is no describing of all possible combinations of the various technical features in the foregoing embodiments. It should be noted that there is no contradiction in the combination of these technical features which should be considered as the scope of the description.

Although present disclosure is illustrated and described herein with reference to specific embodiments, the present disclosure is not intended to be limited to the details shown. It is to be noted that, various modifications may be made in the details within the scope and range of equivalents of the claims and without departing from the present disclosure. Therefore, the protection scope of the present disclosure shall be subject to the protection scope of the claims.

## Claims

1. An atomizer, comprising:
a liquid reservoir (120) having a liquid storage cavity (122) for receiving atomizing liquid, wherein the liquid reservoir (120) has an opening end (121), and the opening end (121) defines an opening in communication with the liquid storage cavity (122);
a heating assembly (130) comprising a liquid conducting body (131) and a heating element (132), wherein
the liquid conducting body (131) is located on the opening end (121),
the conducting body (131) has a liquid absorbing surface (131a) facing an inside of the liquid storage cavity (122) and
an atomizing surface (131b) located outside of the liquid storage cavity (122),
the heating element (132) is formed on the atomizing surface (131b),
the liquid conducting body (131) is configured to conduct the atomizing liquid in the liquid storage cavity (122) to the atomizing surface (131b), and
the heating element (132) is configured to atomize the atomizing liquid conducted to the atomizing surface (131b);
the liquid absorbing surface (131a) defines a recess (131c) in communication with the liquid storage cavity (122),
the recess (131c) is configured for being filled with the atomizing liquid flowed from the liquid storage cavity (122), and
an minimum conduction distance (d3) of the atomizing liquid from a bottom wall of the recess (131c) to the atomizing surface (131b) is less than a minimum conduction distance (d1) of the atomizing liquid from the liquid absorbing surface (131a) to the atomizing surface (131b),
wherein the liquid absorbing surface (131a) and the atomizing surface (131b) are located on opposite sides of the liquid conducting body (131), respectively, and the liquid conducting body (131) further comprises a side surface (131d) connecting the liquid absorbing surface (131a) and the atomizing surface (131b),
wherein the recess extends along a direction from the liquid absorbing surface (131a) to the atomizing surface (131b),
wherein the liquid absorbing surface (131a) and the bottom wall of the recess (131c) are parallel to the atomizing surface (131b).

2. The atomizer according to claim 1, wherein the minimum conduction distance (d3) from the bottom wall of the recess (131c) to the atomizing surface (131b) is less than a depth (d2) of the recess (131c).

3. The atomizer according to claim 1, wherein a cross-sectional area of the recess (131c) gradually decreases along a direction from the liquid absorbing surface (131a) to the atomizing surface (131b).

4. The atomizer according to claim 1, wherein the heating assembly (130) further comprises a sealing element (133), the sealing element (133) is at least partially located between the liquid conducting body (131) and the liquid reservoir (120), so as to prevent the atomizing liquid in the liquid reservoir (120) from directly flowing out without passing through the liquid conducting body (131).

5. The atomizer according to claim 4, wherein a side of the liquid conducting body (131) adjacent to the liquid absorbing surface (131a) extends into the opening of the liquid reservoir (120), the sealing element (133) comprises a sealing body (133a) sleeved on a side surface of the liquid conducting body.

6. The atomizer according to claim 5, wherein the sealing element further comprises a first latching portion (133b) and a second latching portion (133c), the first latching portion (133b) is formed on an end of the sealing body (133a) adjacent to the liquid absorption surface (131a), and the second latching portion (133c) is formed on an end of the sealing body (133a) adjacent to the atomizing surface (131b), and the first latching portion (133b) and the second latching portion (133c) are latched to both ends of the liquid conducting body (131), respectively.

7. The atomizer according to claim 6, wherein the first latching portion (133b) and the second latching portion (133c) are both formed on an inner wall of the sealing body (133a).

8. The atomizer according to claim 5, wherein the sealing element (133) further comprises a third latching portion (133d) formed on an end of an outer wall of the sealing body (133a) adjacent to the atomizing surface (131b).

9. The atomizer according to claim 5, wherein an inner wall of the liquid reservoir (120) provides a strengthening rib (124), and the strengthening rib (124) is capable of abutting against a side of the heating assembly (130) located in the liquid reservoir (120).

10. The atomizer according to claim 1, further comprising a housing (110), wherein the housing (110) defines an air inlet (111) and an air outlet (112), the heating assembly (130) and the liquid reservoir (120) are received in the housing (110), the heating assembly (130) is located adjacent to the air inlet (111), and an airflow passage communicating the air inlet (111) and the air outlet (112) is provided between the housing (110) and the liquid reservoir (120).

11. The atomizer according to claim 10, wherein the heating assembly (130) further comprises a sealing element (133), the sealing element (133) is at least partially located between the liquid conducting body (131) and the liquid reservoir (120), so as to prevent the atomizing liquid in the liquid reservoir (120) from directly flowing out without passing through the liquid conducting body (131); and the sealing element (133) defines a first notch (133f), the opening end (121) defines a second notch (126), and the first notch (133f) and the second notch (126) communicate the air inlet (111) and the airflow passage.

12. An electronic cigarette, comprising: an atomizer (100) according to any one of claims 1 to 11; and a battery device (300) comprising a cover (310), a battery (320), and a pogo pin (330), wherein the battery (320) is received in the cover (310), the pogo pin (330) is mounted on the cover (310), and the pogo pin (330) is electrically connected to the battery (320) and the heating element (132), respectively.

## Patentansprüche

1. Zerstäuber, umfassend:
einen Flüssigkeitsbehälter (120), aufweisend einen Flüssigkeitsspeicherhohlraum (122) zur Aufnahme von Zerstäubungsflüssigkeit, wobei der Flüssigkeitsbehälter (120) ein Öffnungsende (121) aufweist und das Öffnungsende (121) eine Öffnung in Kommunikation mit dem Flüssigkeitsspeicherhohlraum (122) definiert;
eine Heizungsanordnung (130), umfassend einen Flüssigkeitsleitungskörper (131) und ein Heizelement (132), wobei der Flüssigkeitsleitungskörper (131) am Öffnungsende (121) angeordnet ist, der Leitungskörper (131) eine flüssigkeitsabsorbierende Oberfläche (131a) aufweist, die einer Innenseite des Flüssigkeitsspeicherhohlraums (122) zugewandt ist, und eine Zerstäubungsoberfläche (131b), die außerhalb des Flüssigkeitsspeicherhohlraums (122) angeordnet ist, wobei das Heizelement (132) auf der Zerstäubungsoberfläche (131b) geformt ist, der Flüssigkeitsleitungskörper (131) konfiguriert ist, um die Zerstäubungsflüssigkeit in den Flüssigkeitsspeicherhohlraum (122) zur Zerstäubungsoberfläche (131b) zu leiten, und das Heizelement (132) konfiguriert ist, um die zur Zerstäubungsoberfläche (131b) geleitete Zerstäubungsflüssigkeit zu zerstäuben, wobei die flüssigkeitsabsorbierende Oberfläche (131a) eine Vertiefung (131c) in Kommunikation mit dem Flüssigkeitsspeicherhohlraum (122) definiert, wobei die Vertiefung (131c) konfiguriert ist, um mit der vom Flüssigkeitsspeicherhohlraum (122) eingeströmten Zerstäubungsflüssigkeit gefüllt zu werden und ein Mindestleitungsabstand (d3) der Zerstäubungsflüssigkeit von einer Bodenwand der Vertiefung (131c) zur Zerstäubungsoberfläche (131b) geringer ist als ein Mindestleitungsabstand (d1) der Zerstäubungsflüssigkeit von der flüssigkeitsabsorbierenden Oberfläche (131a) zur Zerstäubungsoberfläche (131b, wobei die flüssigkeitsabsorbierende Oberfläche (131a) und die Zerstäubungsoberfläche (131b) jeweils an entgegengesetzten Seiten des Flüssigkeitsleitungskörpers (131) angeordnet sind und der Flüssigkeitsleitungskörper (131) zudem eine Seitenoberfläche (131d) umfasst, die die flüssigkeitsabsorbierende Oberfläche (131a) und die Zerstäubungsoberfläche (131b) miteinander verbindet,
wobei sich die Vertiefung entlang einer Richtung von der flüssigkeitsabsorbierenden Oberfläche (131a) zur Zerstäubungsoberfläche (131b) erstreckt,
wobei die flüssigkeitsabsorbierende Oberfläche (131a) und die Bodenwand der Vertiefung (131c) parallel zur Zerstäubungsoberfläche (131b) angeordnet sind.

2. Zerstäuber nach Anspruch 1, wobei der Mindestleitungsabstand (d3) von der Bodenwand der Vertiefung (131c) zur Zerstäubungsoberfläche (131b) geringer ist als eine Tiefe (d2) der Vertiefung (131c).

3. Zerstäuber nach Anspruch 1, wobei ein Querschnittsbereich der Vertiefung (131c) schrittweise entlang einer Richtung von der flüssigkeitsabsorbierenden Oberfläche (131a) zur Zerstäubungsoberfläche (131b) abnimmt.

4. Zerstäuber nach Anspruch 1, wobei die Heizungsanordnung (130) zudem ein Dichtungselement (133) umfasst, wobei das Dichtungselement (133) mindestens teilweise zwischen dem Flüssigkeitsleitungskörper (131) und dem Flüssigkeitsbehälter (120) angeordnet ist, sodass verhindert wird, dass die Zerstäubungsflüssigkeit im Flüssigkeitsbehälter (120) direkt ausströmt, ohne durch den Flüssigkeitsleitungskörper (131) zu strömen.

5. Zerstäuber nach Anspruch 4, wobei eine Seite des Flüssigkeitsleitungskörpers (131), die an die flüssigkeitsabsorbierende Oberfläche (131a) angrenzt, sich in die Öffnung des Flüssigkeitsbehälters (120) erstreckt, wobei das Dichtungselement (133) einen Dichtungskörper (133a) umfasst, der auf eine Seitenoberfläche des Flüssigkeitsleitungskörpers gestülpt ist.

6. Zerstäuber nach Anspruch 5, wobei das Dichtungselement zudem einen ersten Arretierungsabschnitt (133b) und einen zweiten Arretierungsabschnitt (133c) umfasst, wobei der erste Arretierungsabschnitt (133b) an einem Ende des Dichtungskörpers (133a) angrenzend an die flüssigkeitsabsorbierende Oberfläche (131a) geformt ist und der zweite Arretierungsabschnitt (133c) an einem Ende des Dichtungskörpers (133a) angrenzend an die Zerstäubungsoberfläche (131b) geformt ist und der erste Arretierungsabschnitt (133b) und der zweite Arretierungsabschnitt (133c) jeweils an beiden Enden des Flüssigkeitsleitungskörpers (131) arretiert sind.

7. Zerstäuber nach Anspruch 6, wobei der erste Arretierungsabschnitt (133b) und der zweite Arretierungsabschnitt (133c) beide an einer Innenwand des Dichtungskörpers (133a) geformt sind.

8. Zerstäuber nach Anspruch 5, wobei das Dichtungselement (133) zudem einen dritten Arretierungsabschnitt (133d) umfasst, der an einem Ende einer Außenwand des Dichtungskörpers (133a) angrenzend an die Zerstäubungsoberfläche (131b) geformt ist.

9. Zerstäuber nach Anspruch 5, wobei eine Innenwand des Flüssigkeitsbehälters (120) eine Verstärkungsrippe (124) bereitstellt, und wobei die Verstärkungsrippe (124) in der Lage ist, gegen eine Seite der Heizungsanordnung (130) anzuschlagen, die im Flüssigkeitsbehälter (120) angeordnet ist.

10. Zerstäuber nach Anspruch 1, zudem umfassend ein Gehäuse (110), wobei das Gehäuse (110) einen Lufteinlass (111) und einen Luftauslass (112) definiert, wobei die Heizungsanordnung (130) und der Flüssigkeitsbehälter (120) im Gehäuse (110) aufgenommen sind, die Heizungsanordnung (130) angrenzend an den Lufteinlass (111) angeordnet ist, und ein Luftstromdurchlass den Lufteinlass (111) und den Luftauslass (112) in Kommunikation bringt und zwischen dem Gehäuse (110) und dem Flüssigkeitsbehälter (120) bereitgestellt ist.

11. Zerstäuber nach Anspruch 10, wobei die Heizungsanordnung (130) zudem ein Dichtungselement (133) umfasst, wobei das Dichtungselement (133) mindestens teilweise zwischen dem Flüssigkeitsleitungskörper (131) und dem Flüssigkeitsbehälter (120) angeordnet ist, sodass verhindert wird, dass die Zerstäubungsflüssigkeit im Flüssigkeitsbehälter (120) direkt ausströmt, ohne durch den Flüssigkeitsleitungskörper (131) zu strömen, und
wobei das Dichtungselement (133) eine erste Nut (133f) definiert, wobei das Öffnungsende (121) eine zweite Nut (126) definiert und wobei die erste Nut (133f) und die zweite Nut (126) den Lufteinlass (111) und den Luftstromdurchlass miteinander in Kommunikation bringen.

12. Elektronische Zigarette, umfassend:
einen Zerstäuber (100) nach einem der Ansprüche 1 bis 11 und
eine Batterievorrichtung (300), umfassend eine Abdeckung (310), eine Batterie (320) und einen Federkontaktstift (330), wobei die Batterie (320) in der Abdeckung (310) aufgenommen ist, der Federkontaktstift (330) auf der Abdeckung (310) montiert ist und
der Federkontaktstift (330) elektrisch jeweils mit der Batterie (320) und dem Heizelement (132) verbunden ist.

## Revendications

1. Atomiseur, comprenant :
un réservoir de liquide (120) comportant une cavité de stockage (122) de liquide pour recevoir un liquide d'atomisation, dans lequel le réservoir de liquide (120) comporte une extrémité d'ouverture (121), et l'extrémité d'ouverture (121) définit une ouverture en communication avec la cavité de stockage (122) de liquide ;
un ensemble chauffant (130) comprenant un corps conducteur (131) de liquide et un élément chauffant (132), dans lequel le corps conducteur (131) de liquide est situé sur l'extrémité d'ouverture (121), le corps conducteur (131) comporte une surface d'absorption (131a) de liquide faisant face à un intérieur de la cavité de stockage (122) de liquide et à une surface d'atomisation (131b) située à l'extérieur de la cavité de stockage (122) de liquide, l'élément chauffant (132) est formé sur la surface d'atomisation (131b), le corps conducteur (131) de liquide est configuré pour acheminer le liquide d'atomisation dans la cavité de stockage (122) de liquide vers la surface d'atomisation (131b), et l'élément chauffant (132) est configuré pour pulvériser le liquide d'atomisation acheminée vers la surface d'atomisation (131b) ; la surface d'absorption (131a) de liquide définit un renfoncement (131c) en communication avec la cavité de stockage (122) de liquide, le renfoncement (131c) est configuré pour être rempli avec le liquide d'atomisation s'écoulant de la cavité de stockage (122) de liquide,
et une distance d'acheminement minimale (d3) du liquide d'atomisation d'une paroi inférieure du renfoncement (131c) à la surface d'atomisation (131b) est inférieure à une distance d'acheminement minimale (d1) du liquide d'atomisation de la surface d'absorption (131a) de liquide vers la surface d'atomisation (131b),
dans lequel la surface d'absorption (131a) de liquide et la surface d'atomisation (131b) sont situées sur des côtés opposés du corps conducteur (131) de liquide, respectivement, et le corps conducteur (131) de liquide comprend de plus une surface latérale (131d) reliant la surface d'absorption (131a) de liquide et la surface d'atomisation (131b),
dans lequel le renfoncement se prolonge le long d'une direction allant de la surface d'absorption (131a) de liquide à la surface d'atomisation (131b),
dans lequel la surface d'absorption (131a) de liquide et la paroi inférieure du renfoncement (131c) sont parallèles à la surface d'atomisation (131b).

2. Atomiseur selon la revendication 1, dans lequel la distance d'acheminement minimale (d3) de la paroi inférieure du renfoncement (131c) et la surface d'atomisation (131b) est inférieure à une profondeur (d2) du renfoncement (131c).

3. Atomiseur selon la revendication 1, dans lequel une surface à section transversale du renfoncement (131c) diminue progressivement dans une direction allant de la surface d'absorption (131a) de liquide vers la surface d'atomisation (131b).

4. Atomiseur selon la revendication 1, dans lequel l'ensemble chauffant (130) comprend de plus un élément d'étanchéité (133), l'élément d'étanchéité (133) est au moins partiellement situé entre le corps conducteur (131) de liquide et le réservoir de liquide (120) de manière à empêcher le liquide d'atomisation dans le réservoir de liquide (120) de s'écouler directement vers l'extérieur sans passer par le corps conducteur de liquide (131).

5. Atomiseur selon la revendication 4, dans lequel un côté du corps conducteur (131) de liquide adjacent à la surface d'absorption (131a) de liquide se prolonge dans l'ouverture du réservoir de liquide (120), l'élément d'étanchéité (133) comprend un corps d'étanchéité (133a) chemisé sur une surface latérale du corps conducteur de liquide.

6. Atomiseur selon la revendication 5, dans lequel l'élément d'étanchéité comprend de plus une première partie de verrouillage (133b) et une seconde partie de verrouillage (133c), la première partie de verrouillage (133b) est formée sur une extrémité du corps d'étanchéité (133a) adjacente à la surface d'absorption (131a) de liquide, et la seconde partie de verrouillage (133c) est formée sur une extrémité du corps d'étanchéité (133a) adjacente à la surface d'atomisation (131b), et la première partie de verrouillage (133b) et la seconde partie de verrouillage (133c) sont, respectivement, verrouillées aux deux extrémités du corps conducteur de liquide (131).

7. Atomiseur selon la revendication 6, dans lequel la première partie de verrouillage (133b) et la seconde partie de verrouillage (133c) sont toutes deux formées sur une paroi intérieure du corps d'étanchéité (133a).

8. Atomiseur selon la revendication 5, dans lequel l'élément d'étanchéité (133) comprend de plus une troisième partie de verrouillage (133d) formée sur une extrémité d'une paroi extérieure du corps d'étanchéité (133a) adjacente à la surface d'atomisation (131b).

9. Atomiseur selon la revendication 5, dans lequel une paroi intérieure du réservoir de liquide (120) fournit une nervure de renforcement (124), et la nervure de renforcement (124) est en mesure de se mettre en butée contre un côté de l'ensemble chauffant (130) situé dans le réservoir de liquide (120).

10. Atomiseur selon la revendication 1, comprenant de plus un logement (110), dans lequel le logement (110) définit une entrée d'air (111) et une sortie d'air (112), l'ensemble chauffant (130) et le réservoir de liquide (120) sont reçus dans le logement (110), l'ensemble chauffant (130) est situé de manière adjacente à l'entrée d'air (111), et un passage de flux d'air faisant communiquer l'entrée d'air (111) et la sortie d'air (112) est prévu entre le logement (110) et le réservoir de liquide (120).

11. Atomiseur selon la revendication 10, dans lequel l'ensemble chauffant (130) comprend de plus un élément d'étanchéité (133), l'élément d'étanchéité (133) est au moins partiellement situé entre le corps conducteur (131) de liquide et le réservoir de liquide (120), de manière à empêcher le liquide d'atomisation dans le réservoir de liquide (120) de s'écouler directement vers l'extérieur sans passer par le corps conducteur (131) de liquide ; et
l'élément d'étanchéité (133) définit une première encoche (133f), l'extrémité d'ouverture (121) définit une seconde encoche (126), et la première encoche (133f) et la seconde encoche (126) font communiquer l'entrée d'air (111) et le passage du flux d'air.

12. Cigarette électronique, comprenant :
un atomiseur (100) selon l'une quelconque des revendications 1 à 11 ; et
un dispositif de batterie (300) comprenant un couvercle (310), une batterie (320) et une goupille creuse (330), dans lequel la batterie (320) est reçue dans le couvercle (310), la goupille creuse (330) est montée sur le couvercle (310) et la goupille creuse (330) est, respectivement, connectée électriquement à la batterie (320) et à l'élément chauffant (132).
